# EUROPEAN PATENT APPLICATION

(11) **EP 1 864 963 A1**
(43) Date of publication of application: **12.12.2007**
(21) Application number: 06011437.8
(22) Date of filing: 02.06.2006
(51) Int. Cl.: C07C 67/30, C07C 69/614, C09K 15/08, C08K 5/134, A61K 8/37

(54) **Hydroxy-aromatic compound, process for its preparation, and use as antioxidant**

(71) Applicant: DSM IP Assets B.V., 6411 TE Heerlen (NL)
(72) Inventor: Van Benthem, Rudolfus Antonius Theodorus Maria, 6141 BR Limbricht (NL); Gijsman, Pieter, 6191 NM Beek (NL)
(74) Representative: Koster, Nico

(57) **Abstract**

The invention relates to a process for the preparation of a hydroxy-aromatic compound. The process comprises the steps of bringing a starting compound according to formula (I) together with an alkanol hemiacetal according to formula (II) to form a reaction mixture. The reaction mixture is then brought to conditions whereby the hydroxy-aromatic compound is formed. Formula (I) is: 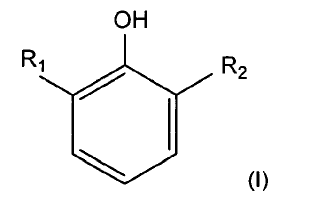 wherein R₁ and R₂ each individually are a C₁ - C₂₀ alkyl group. Formula (II) is: 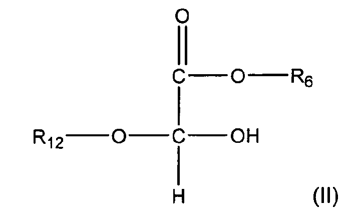 wherein R₆ is a C₁-C₁₂ alkyl group, aryl group, aralkyl group or cycloalkyl group and wherein R₁₂ is H, a C₁-C₁₂ alkyl group, aryl group, aralkyl group or cycloalkyl group.

## Description

The invention relates to a process for the preparation of a hydroxy-aromatic compound; the invention further relates to hydroxy-aromatic compounds and to their use.

Hydroxy-aromatic compounds as such are known, and are herein defined as compounds having an aromatic ring with at least one -OH group attached directly to it. Certain classes of hydroxy-aromatic compounds have radical-scavenging ability and are for this reason widely used as antioxidant in various compositions, a.o. in thermoplastic polymers or in body care products. A typical example of a hydroxy-aromatic antioxidant is the well-known compound Irganox™ 1300 - see formula below, wherein Me refers to a methyl group:

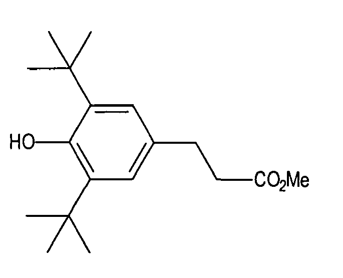

A disadvantage of the known hydroxy-aromatic antioxidants is that their radical-scavenging function per molecule is rather limited; furthermore, the service life and heat resistance of known hydroxy-aromatic antioxidants is often less than optimal.

It is the objective of the present invention to reduce the abovementioned disadvantages.

The said objective is achieved by a hydroxy-aromatic compound according to formula (III): 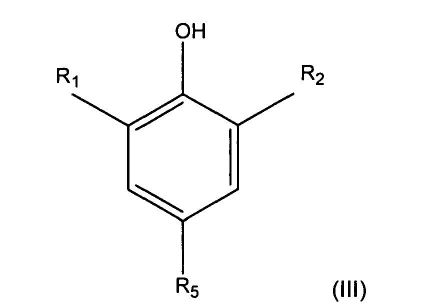 wherein R₁ and R₂ each individually are a C₁ - C₂₀ alkyl group and wherein R₅ is a group according to formula (IV): 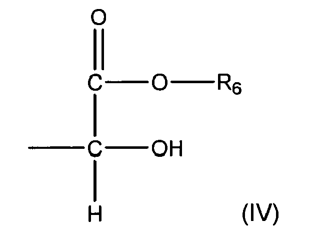 wherein R₆ is is a C₁-C₁₂ alkyl group, aryl group, aralkyl group or cycloalkyl group.

An advantage of the hydroxy-aromatic compound according to the invention is that the R₅ group contributes more strongly to radical-scavenging activity than the comparable group in the known antioxidants.

The invention further relates to a process for the preparation of hydroxy-aromatic compounds according to formula (III), comprising the steps of:
- bringing a starting compound according to formula (I) together with an alkanol hemiacetal according to formula (II) to form a reaction mixture, wherein:
   o formula (I) is: 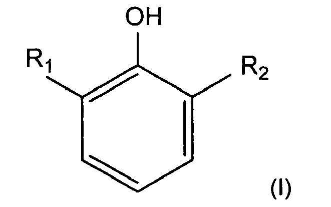 wherein R₁ and R₂ each individually are a C₁ - C₂₀ alkyl group;
   o formula (II) is: 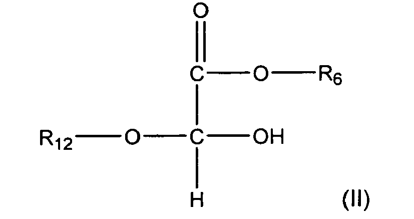 wherein R₆ is a C₁-C₁₂ alkyl group, aryl group, aralkyl group or cycloalkyl group and wherein R₁₂ is H, a C₁-C₁₂ alkyl group, aryl group, aralkyl group or cycloalkyl group;
- bringing the reaction mixture to conditions whereby the hydroxy-aromatic compound is formed.

The process according to the invention comprises the step of bringing raw materials together to form a reaction mixture. One of the said raw materials is the starting compound of formula (I). The starting compound is itself a hydroxy-aromatic compound. Characteristic of the starting compound is that it is ortho-substituted, as indicated in formula (I) by R₁ and R₂. R₁ and R₂ may be the same or they may be different, and are a C₁ - C₁₂ alkyl group. In a preferred embodiment, R₁ and R₂ are both tert-butyl or both methyl, or R₁ is tert-butyl and R₂ is methyl; these compounds are as such known. The compound according to formula (I) may be one single compound but is understood to also comprise the meaning of a mixture of two or more compounds falling within the scope of the formulas as defined above. Examples of preferred compounds according to formula (I) are (2, 6-)di-tert-butylphenol, (2,6-)dimethylphenol and 2-tert-butyl-6-methyl-phenol.

Aside from the compound according to formula (I), the starting materials for the process according to the invention also comprise an alkanol hemiacetal of formula (II). In formula (II), R₆ is a C₁-C₁₂ alkyl group, aryl group, aralkyl group or cycloalkyl group and R₁₂ is H, a C₁-C₁₂ alkyl group, aryl group, aralkyl group or cycloalkyl group. Preferably R₆ and R₁₂ are C₁-C₁₂ alkyl groups. Examples thereof are methyl, ethyl, propyl, butyl, pentyl, hexyl, and heptyl. R₆ and R₁₂ are in particular a methyl group or an ethyl group. The compound according to formula (II) may be one single compound but is understood to also comprise the meaning of a mixture of two or more compounds falling within the scope of the formulas as defined above. Examples of preferred compounds according to formula (II) are methylglyoxylate methanol hemiacetal (GMHA™ , DSM Fine Chemicals, Linz); ethylglyoxylate ethanol hemiacetal (GEHA™, DSM Fine Chemicals, Linz); ethylglyoxylate methanol hemiacetal; butylglyoxylate butanol hemiacetal; butylglyoxylate methanol hemiacetal; butylglyoxylate ethanol hemiacetal; isopropylglyoxylate isopropanol hemiacetal; propylglyoxylate propanol hemiacetal; cyclohexylglyoxylate methanol hemiacetal and 2-ethylhexylglyoxylate methanol hemiacetal.

The bringing together of the raw materials to form the reaction mixture may be accomplished by simply mixing them; it may be beneficial to do this in the presence of a solvent. It may thus be beneficial to execute the reaction step according to the invention in a solvent or dispersant. As solvents, those compounds are suitable in which the reactants dissolve sufficiently to let the reaction take place. Examples of such solvents are water and various organic solvents. Depending on the specific compound or compounds of formula (I) and (II), it may well be possible to use one or more of the reactants as solvent; in such a case, it can be possible to forego on the use of a solvent that is essentially a non-reactant and to execute the reaction step in bulk. In particular, many of the compounds according to formula (II) are a liquid at temperatures between 10°C and 100°C and can act as dispersant/solvent as well as reactant.

Once the reaction mixture is formed, it should be brought to conditions whereby the hydroxy-aromatic compound of formula (III) can be formed, i.e. in a reaction step. Although the reaction step may proceed spontaneously once the respective compounds have been brought together, it may be useful to bring the compounds together in the presence of a catalyst in order to accelerate the reaction. As catalyst, preferably an acid is used; in particular, a Lewis or a Bronsted type of acid is preferred - such as for example sulphuric acid - whereby the pH is reduced to between 0 and 5, preferably to between 1 and 4, in particular to between 2 and 3. Suitable examples of acid catalysts are sulphuric acid, nitric acid, hydrochloric acid, phosphoric acid, boric acid, tetrafluoroboric acid, paratoluene sulphonic acid, formic acid, ammonium sulphate, ammonium chloride, ammonium nitrate.

A preferred embodiment of the hydroxy-aromatic compound according to the invention is the compound of formula (V): 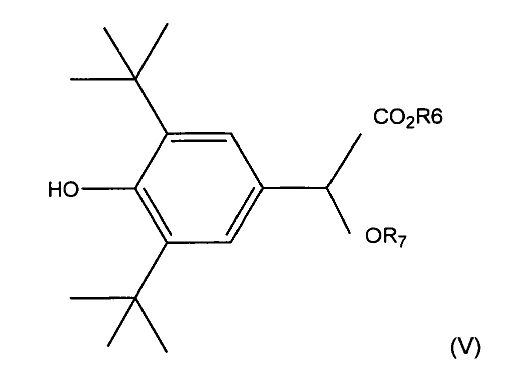 The term R₆ in formula (V) refers to a C₁-C₁₂ alkyl group, aryl group, aralkyl group or cycloalkyl group. Preferably, R₆ is methyl. In Formula (V), R₇ is H or an alkyl, or -OR₇ is a hydroxy group. Preferably, R₇ is H or -OR₇ is a hydroxy-aromatic group such that it is the compound of formula (VI), which represents another preferred embodiment of the invention:

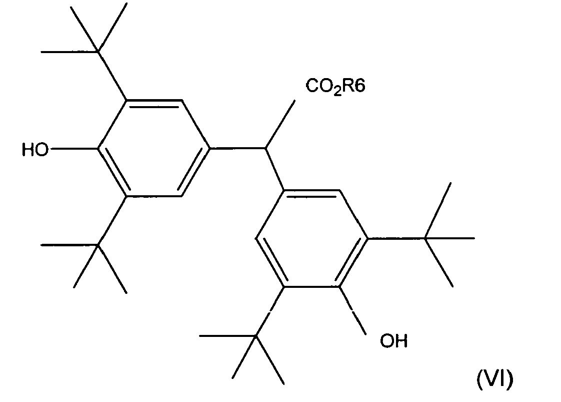

The molar ratio between the raw materials that are brought together in the reaction mixture may vary between wide limits. The molar ratio between the alkanol hemiacetal compound of formula (II) (A) and the starting compound of formula (I) (H), herein referred to as the A/H ratio, preferably lies between about 0.1 and about 10. The molar ratio A/H is preferably at least 0.1, 0.2, 0.3, 0.4, 0.5, 0.6, 0.7 or 0.8, and preferably at most 10, 9, 8, 7, 6, 5, 4, 3, 2, 1.5 or 1.2.

If the molar A/H ratio lies above 1, then this will generate a preference towards the formation of compounds of formula (V). If the molar A/H ratio lies below 1, then this will generate a preference towards the formation of compounds of formula (VI). It was furthermore found that a preference towards the formation of compounds of formula (V) rather than compounds of formula (VI) may be strengthened by choosing lower temperatures and less acidic catalysts.

In certain uses, it may be desirable to decrease the volatility of hydroxy-aromatic compounds according to the invention; this may occur in for example the preparation of polymer compositions comprising a hydroxy-aromatic compound according to the invention. A method of achieving the said reduction of volatility is by implementing an increase in molecular weight. The said increase in molecular weight should be accompanied by a minimal or even no reduction of hydroxy-aromatic functionality. This may be achieved by implementing a transesterification step, which is preferably base-catalysed. In a preferred embodiment, transesterification is done by using a multi-functional ester such as pentaerythritol. In another preferred embodiment, transesterification is done by using an ester having an alkyl group of more than 12 carbon atoms, such as stearyl alcohol. It is an advantage of the hydroxy-aromatic compounds according to the invention that the ester functionality that is already present via the '-CO₂R₆' group may be favourably employed to implement the modification step via transesterification.

In an alternative embodiment of the invention, the process for preparation of the hydroxy-aromatic compound is carried out by using as starting compound a compound of formula (I) wherein R₁ is a C₁-C₂₀ group and preferably a methyl or tert-butyl group and wherein R₂ is H. Thus, in this embodiment the hydroxy-aromatic starting compound is ortho-substituted only once. In this embodiment, it is preferred that the A/H ratio lies between 1.3 and 1.7 and is preferably essentially 1.5. This has - in combination with the preferred choices of tert-butyl for R₁ and methyl for R₆ - the advantage that a preference is generated for the formation of a hydroxy-aromatic compound of formula (VII):

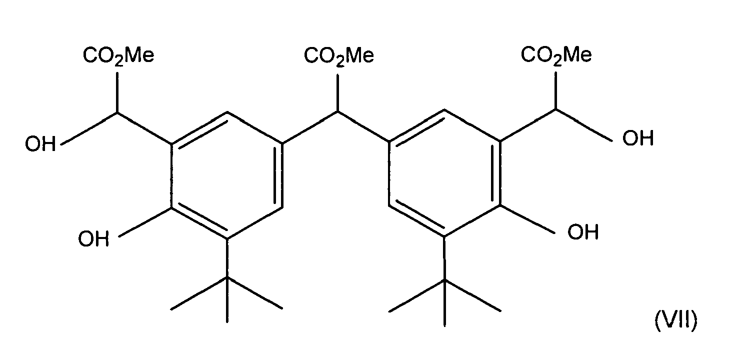

The hydroxy-aromatic compound according to formula (III) - and thus also according to formulas (V), (VI) and (VII) - of the invention is suitable for a variety of uses, whereby one of the preferred uses is the use as antioxidant. The use as antioxidant is in particular preferred in thermoplastic or thermosetting polymers or in personal care compositions. The said use may comprise the direct addition of the hydroxy-aromatic compound according to the invention to a polymer or personal care composition. It may, however, be beneficial to first create a stabilising composition containing the hydroxy-aromatic compound of the invention and then add the stabilising composition to a polymer or to a personal care composition. The invention thus also relates to a stabilising composition comprising between 0.05 wt.% and 15 wt.% of the hydroxy-aromatic compound of formula (III) and, optionally, other usual additives.

Thermoplastics in which the hydroxy-aromatic compound according to the invention may be suitable used include polyolefinic thermoplastics such as polyethylene and polypropylene, polyesters, polyurethanes, polyamides, and polycarbonates. In the case of the specific embodiment of the invention of the hydroxy-aromatic compound of formula (VII), the possibility arises that the said compound of formula (VII) has a double use: it may be used as a co-monomer - by using the aliphatic hydroxy groups - and at the same time exercise an anti-oxidant function via the aromatic hindered hydroxy groups. The said double use is particularly advantageous in polyurethanes: the compound of formula (VII) acts as the diol - or as one of the diols - that is brought into contact with di-isocyanate compounds in known fashion, leading to the formation of a polyurethane.

The term personal care composition as used herein encompasses body-care compositions such as:
- skin care preparations such as body oils, body lotions, body gels, treatment creams, skin protection ointments, shaving preparations, skin powders, moisturizing gels, moisturizing sprays, anti-cellulite gels, anti-acne preparations and peeling preparations;
- hair-care preparations such as shampoo, hair conditiones, products for styling and treating hair, perming agents, hair sprays and lacquers, hair gels;
- deodorants and anti-perspirants;
- decorative preparations such as lipstick, nail varnishes, eye shadow, mascaras, dry and moist make-up, rouge, powders, depilatory agents, suntan lotions.

Body care compositions in accordance with the invention such as cosmetic and dermatological compositions can be in the form of a liquid, lotion, a thickened lotion, a gel, a cream, a milk, an ointment, a paste, a powder, or a solid tube stick and can be optionally be packaged as an aerosol and can be provided in the form of a mousse, foam or a spray foams, sprays, sticks or aerosols or wipes.

The body care compositions according to the invention can be in the form of a suspension or dispersion in solvents or fatty substances, or alternatively in the form of a (micro)emulsion - such as emulsions of the oil-in-water type (O/W) or water-in-oil (W/O) type - such as a cream or a milk, a vesicular dispersion, in the form of an ointment, a gel, a solid tube stick or an aerosol mousse. The emulsions can also contain anionic, nonionic, cationic or amphoteric surfactants.

If the hydroxy-aromatic compound or the stabilizing composition according to the invention is or are present in an emulsion, they may be present in the continuous or in the discontinuous phase of the emulsion.

A stabilising composition is preferably used for the stabilising of colorants, dyes, scents, fragrances, active ingredients or mixtures thereof in body care products and household products. Typically, a use of the stabilising composition is preferred such that the product itself - e.g, the household product or body care product - contains between 0.01 wt.% and 5 wt.% of a hydroxy-aromatic compound according to the invention.

## Claims

1. Process for the preparation of a hydroxy-aromatic compound, comprising the steps of:
• bringing a starting compound according to formula (I) together with an alkanol hemiacetal according to formula (II) to form a reaction mixture, wherein:
o formula (I) is: 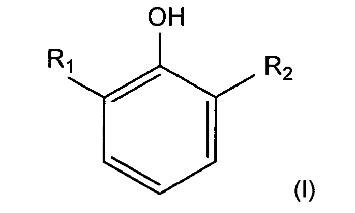 wherein R₁ and R₂ each individually are a C₁ - C₂₀ alkyl group;
o formula (II) is: 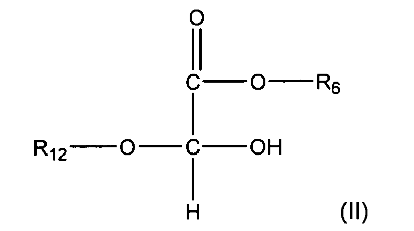 wherein R₆ is a C₁-C₁₂ alkyl group, aryl group, aralkyl group or cycloalkyl group and wherein R₁₂ is H, a C₁-C₁₂ alkyl group, aryl group, aralkyl group or cycloalkyl group;
• bringing the reaction mixture to conditions whereby the hydroxy-aromatic compound is formed.

2. Process according to claim 1, wherein R₁ and R₂ are both tert-butyl or both methyl, or wherein R₁ is tert-butyl and R₂ is methyl.

3. Hydoxy-aromatic compound according to formula (III): 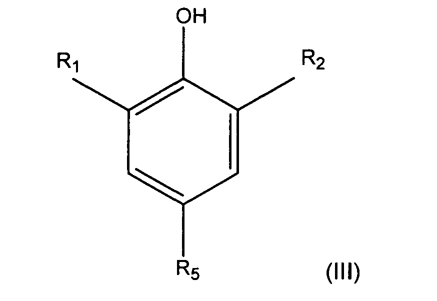 wherein R₁ and R₂ each individually are a C₁ - C₂₀ alkyl group and wherein R₅ is a group according to formula (IV) 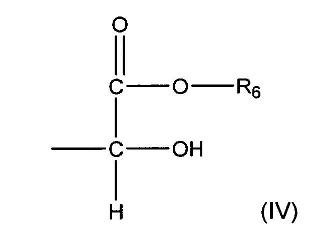 wherein R₆ is is a C₁-C₁₂ alkyl group, aryl group, aralkyl group or cycloalkyl group.

4. Hydroxy-aromatic compound according to claim 3, wherein R₁ and R₂ are both tert-butyl or both methyl, or wherein R₁ is tert-butyl and R₂ is methyl, and wherein R₆ is methyl.

5. Use of the hydroxy-aromatic compound according to claim 3 or 4 as antioxidant.

6. Use of the hydroxy-aromatic compound according to claim 3 or 4 as antioxidant in polymers or in personal care compositions.

7. Stabilising composition comprising between 0.05 wt.% and 15 wt.% of the hydroxy-aromatic compound according to claim 3 or 4 and, optionally, other usual additives.

8. Hydroxy-aromatic compound of formula (VII): 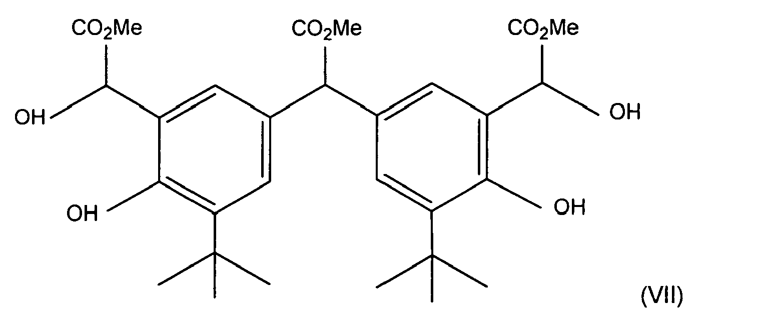 wherein Me is methyl.

9. Use of the hydroxy-aromatic compound of claim 8 as monomer or co-monomer in the preparation of a polymer, in particular polyurethane.

10. Stabilising composition according to claim 7, wherein the composition is used in thermoplastic or thermosetting polymers.

11. Use of a stabilising composition according to claim 7 for the stabilising of colorants, dyes, scents, fragrances, active ingredients or mixtures thereof in body care products and household products.

12. Body care product or thermoplastic polymer or thermosetting polymer, comprising between 0.01 wt.% and 5 wt.% of the hydroxy-aromatic compound according to claim 3 or 4 or 8.
